# EUROPEAN PATENT APPLICATION

(11) **EP 4 122 433 A1**
(43) Date of publication of application: **25.01.2023**
(21) Application number: 20925706.2
(22) Date of filing: 24.12.2020
(51) Int. Cl.: A61F 7/00, A61F 7/10

(54) **COLD COMPRESS CIRCULATING DEVICE AND COLD COMPRESS CIRCULATING SYSTEM**

(30) Priority: 18.03.2020 CN 202010192944
(71) Applicant: Suzhou Microport Rehabtech (Group) Co., Ltd., Suzhou, Jiangsu 215000 (CN)
(72) Inventor: RUAN, Jiantao, Suzhou, Jiangsu 215000 (CN); DI, Pei, Suzhou, Jiangsu 215000 (CN); YANG, Yiwei, Suzhou, Jiangsu 215000 (CN); XIE, Fei, Suzhou, Jiangsu 215000 (CN)
(74) Representative: Lavoix
(86) International application number: PCT/CN2020/139171
(87) International publication number: WO 2021/184871

(57) **Abstract**

A cold compress circulation device (10) and a cold compress circulation system are disclosed. The cold compress circulation device (10) includes a casing (100), a water reservoir (110), a cooler (120), an outlet pipe (130), a return pipe (140) and an ice box (150). Both the water reservoir (110) and the cooler (120) are arranged in the casing (100), and at least part of the ice box (150) is detachably arranged in the water reservoir (110). Both the outlet pipe (130) and the return pipe (140) are brought into fluidic communication with the water reservoir (110), and the cooler (120) is arranged on the outlet pipe (130) and/or the return pipe (140). In this arrangement, the detachable ice box (150) serves as a means for accelerated cooling and can be conveniently replaced by a user. Moreover, the ice box (150) does not lead to an increased amount of water to be circulated or release small ice piece when heated, which may lead to reduced cooling efficiency. Thus, both enhanced cooling efficiency and increased convenience of use are achieved for the user.

## Description

### TECHNICAL FIELD

The present invention relates to the technical field of medical apparatus and, in particular, to a cold compress circulating device and a cold compress circulating system.

### BACKGROUND

In the current medical practice, cold/hot compress is often used to therapeutically treat part of the body of a patient. However, existing circulation devices for cold/hot compress have insufficient cooling efficiency. In order to achieve higher cooling efficiency, some of the apparatus are supplied with ice. However, this approach is prone to the generation of small ice pieces which may block a pipeline and lead to even lower cooling efficiency. Moreover, the supply of ice can cause inconvenience to a user.

### SUMMARY OF THE INVENTION

It is an object of the present invention to provide a cold compress circulation device and a cold compress circulation system, which solve the problems of low cooling efficiency and inconvenient use of conventional cooling devices.

To this end, according to one aspect of the present invention, there is provided a cold compress circulation device, comprising a casing, a water reservoir, a cooler, an outlet pipe, a return pipe and an ice box,
both the water reservoir and the cooler arranged in the casing, at least part of the ice box detachably arranged in the water reservoir, both the outlet pipe and the return pipe in fluidic communication with the water reservoir, the cooler arranged on the outlet pipe and/or the return pipe.

Optionally, the water reservoir may comprise a water reservoir body and an accommodating cavity at the bottom of the water reservoir body, wherein the at least part of the ice box is detachably inserted into the accommodating cavity.

Optionally, the accommodating cavity may comprise a stopper having an inner radial dimension that is smaller than an outer radial dimension of the ice box in order to limit a movement of the ice box.

Optionally, the stopper may comprise at least two protrusions arranged circumferentially, and wherein any two of the protrusions are separated apart by a distance that is smaller than the outer radial dimension of the ice box.

Optionally, the stopper may comprise an annular protrusion extending along the entire circumference of the ice box, and wherein the protrusion has an inner radial dimension that is smaller than the outer radial dimension of the ice box.

Optionally, the return pipe may be connected to the accommodating cavity, a gap is formed between the accommodating cavity and the ice box, and the water reservoir body is brought into communication with the return pipe by the gap.

Optionally, the accommodating cavity may have an elliptic or polygonal cross section, with the ice box having a circular cross section.

Optionally, the ice box may enclose therein a cooling gel or another liquid configured for heat exchange, and the cooling gel has a specific heat capacity greater than water.

Optionally, the cooler may comprise a semiconductor cooling device and a cooling fan, the semiconductor cooling device comprising a cooling surface and a heat dissipation surface, wherein the cooling surface is thermally conductively connected to the outlet pipe or the return pipe, and the cooling fan is configured to dissipate heat from the heat dissipation surface of the semiconductor cooling device.

Optionally, heat dissipation holes may be provided in a portion of the casing corresponding to the cooling fan.

Optionally, the cooler may comprise a micro-compressor.

Optionally, a fluid injection port may be provided on the top of the casing to provide an access to the water reservoir, wherein the ice box is inserted into the water reservoir through the fluid injection port.

Optionally, the casing may comprise a lid which can be opened by removing it or pushing or pulling it aside, wherein the ice box is inserted into the water reservoir when the lid is removed or pushed or pulled aside.

Optionally, the cold compress circulation device may further comprise an air pump and a water pump both arranged in the casing, the air pump and the water pump fluidically independent of each other, the water pump comprising an inlet port arranged at the bottom of the water reservoir body, the inlet port of the water pump oriented vertically and directly connected to the bottom of the water reservoir body, the water pump having an outlet port oriented horizontally and connected to the cooler or the outlet pipe.

Optionally, the cold compress circulation device may further comprise an outlet interface, a return interface and a pressurization interface disposed between the above two, wherein the outlet interface, the return interface and the pressurization interface are all arranged on the casing, the outlet interface connected to the outlet pipe, the return interface connected to the return pipe, the pressurization interface connected to the air pump.

Optionally, the pressurization interface may be disposed between the outlet interface and the return interface.

Optionally, the ice box may be disposable or reusable and enclose therein a liquid configured for heat exchange.

According to another aspect of the present invention, there is provided a cold compress circulation system, comprising the cold compress circulation device as defined above and a pack, the pack brought into fluidic communication with the cold compress circulation device by a connection pipe.

Optionally, the pack may be detachable or fixedly connected to the connection pipe.

Optionally, at least two of the pack, the connection pipes and the cold compress circulation device may be integral or pluggable with each other.

In summary, the present invention provides a cold compress circulation device and a cold compress circulation system. The cold compress circulation device includes a casing, a water reservoir, a cooler, an outlet pipe, a return pipe and an ice box. Both the water reservoir and the cooler are arranged in the casing, and at least part of the ice box is detachably arranged in the water reservoir. Both the outlet and return pipes are brought into fluidic communication with the water reservoir, and the cooler is arranged on the outlet pipe and/or the return pipe. In this arrangement, the detachable ice box serves as a means for accelerated cooling and can be conveniently replaced by a user. Moreover, the ice box does not lead to an increased amount of water to be circulated or release small ice piece when heated, which may lead to reduced cooling efficiency. Thus, both enhanced cooling efficiency and increased convenience of use are achieved for the user.

### BRIEF DESCRIPTION OF THE DRAWINGS

Those of ordinary skill in the art would appreciate that the following drawings are presented merely to enable a better understanding of the present invention rather than to limit the scope thereof in any sense. In the drawings:
Fig. 1 is an overall schematic diagram of a cold compress circulation system according to an embodiment of the present invention;
Fig. 2 is a schematic interior view of a cold compress circulation device according to an embodiment of the present invention;
Fig. 3 is a schematic cross-sectional view of the cold compress circulation device according to an embodiment of the present invention;
Fig. 4 is a schematic interior view of the cold compress circulation device from another perspective according to an embodiment of the present invention;
Fig. 5 is a schematic cross-sectional view of the cold compress circulation device from another perspective according to an embodiment of the present invention;
Fig. 6 is a front view of the cold compress circulation device according to an embodiment of the present invention;
Fig. 7 is a top view of the cold compress circulation device according to an embodiment of the present invention;
Fig. 8 is a schematic diagram of a stopper according to an embodiment of the present invention;
Fig. 9 is a schematic diagram showing an example of cross sections of an accommodating cavity and an ice box according to an embodiment of the present invention; and
Fig. 10 is a schematic diagram showing another example of cross sections of an accommodating cavity and an ice box according to an embodiment of the present invention.

### List of Reference Numerals:

10: Cold Compress Circulation Device; 100: Casing; 110: Water Reservoir;
111: Ice Box Receptacle; 112: Water Reservoir Body; 113: Accommodating Cavity;
114: Stopper; 120: Cooler; 121: Semiconductor Cooling Device; 122: Cooling Fan;
130: Outlet Pipe; 140: Return Pipe; 150: Ice Box; 160: Water Pump; 170: Pipe
Interface; 180: Air Pump; 191: Display Screen; 192: Handle;
20: Pack; 21: Connection Pipe.

### DETAILED DESCRIPTION

Objects, features and advantages of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear despription of the embodiments. In addition, the structures shown in the figures are usually partially representations of their actual counterparts. In particular, as the figures would have different emphases, they are sometimes drawn to different scales.

As used herein, the singular forms "a", "an" and "the" include plural referents, unless the context clearly dictates otherwise. As used herein, the term "or" is generally employed in the sense of "and/or", and "several" of "at least one", unless the context clearly dictates otherwise.

The present invention seeks primarily to solve the problems of low cooling efficiency and inconvenient use of conventional cooling devices by presenting a cold compress circulation device and a cold compress circulation system.

The following description is set forth with reference to the accompanying drawings.

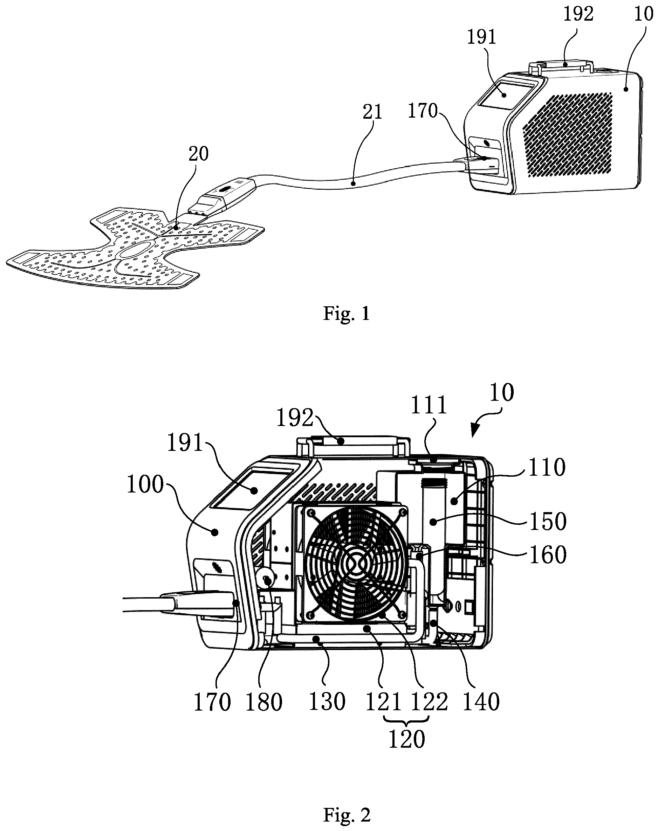

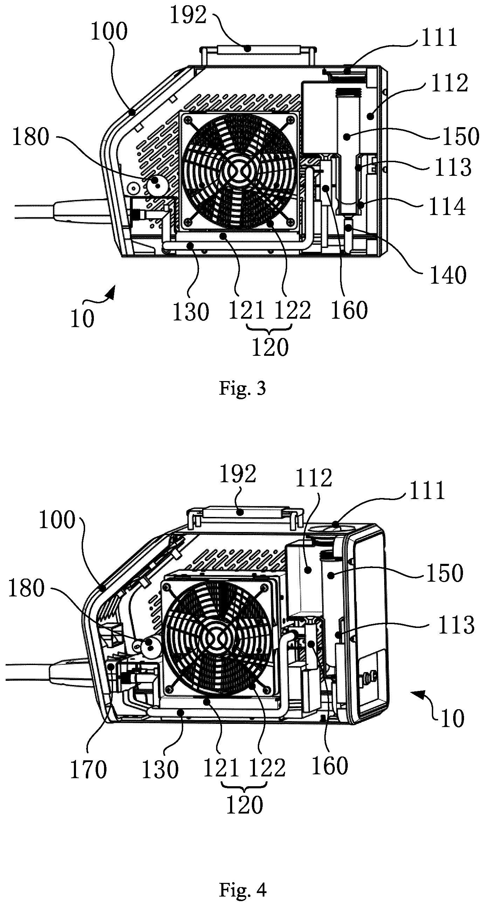

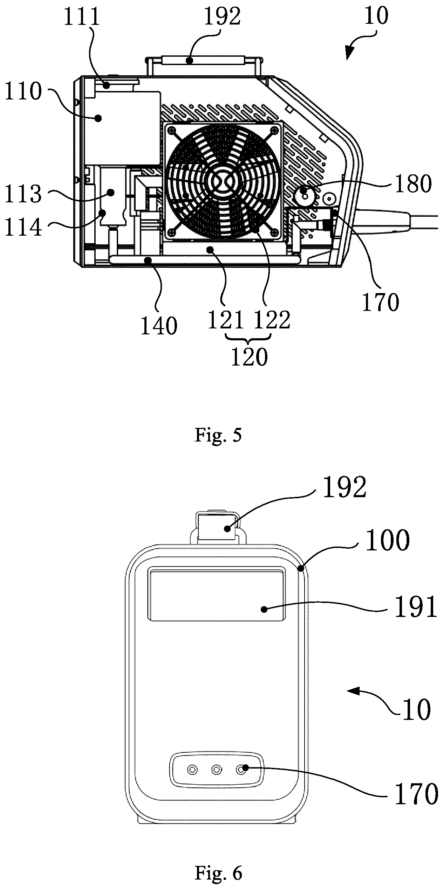

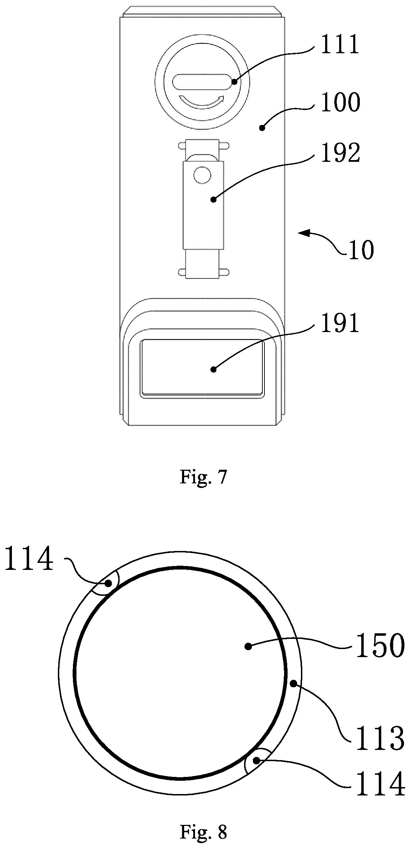

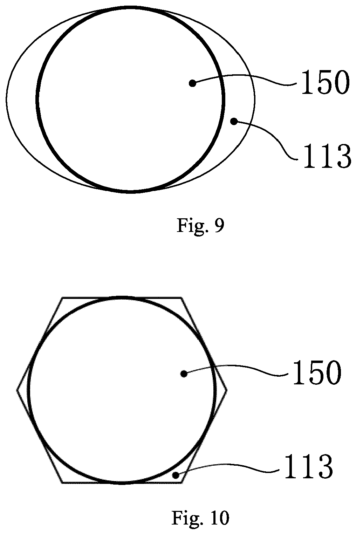

Reference will now be made to Figs. 1-10. Fig. 1 is an overall schematic diagram of a cold compress circulation system according to an embodiment of the present invention. Fig. 2 is a schematic diagram showing the internal structure of a cold compress circulation device according to an embodiment of the present invention. Fig. 3 is a schematic cross-sectional view of a cold compress circulation device according to an embodiment of the present invention. Fig. 4 is a schematic interior view of the cold compress circulation device from another perspective according to an embodiment of the present invention. Fig. 5 is a schematic cross-sectional view of the cold compress circulation device from another perspective according to an embodiment of the present invention. Fig. 6 is a front view of the cold compress circulation device according to an embodiment of the present invention. Fig. 7 is a top view of the cold compress circulation device according to an embodiment of the present invention. Fig. 8 is a schematic diagram of a stopper according to an embodiment of the present invention. Fig. 9 is a schematic diagram showing an example of cross sections of an accommodating cavity and an ice box according to an embodiment of the present invention. Fig. 10 is a schematic diagram showing another example of cross sections of an accommodating cavity and an ice box according to an embodiment of the present invention.

As shown in Fig. 1, an embodiment of the present invention provides a cold compress circulation system intended for use for therapeutic treatment of part of a patient's body by cold compress. The cold compress circulation system includes a cold compress circulation device 10 and a pack 20. The cold compress circulation device 10 is adapted primarily to supply cooling water to the pack 20. The pack 20 is adapted to wrap a diseased part to enable a therapeutic treatment thereon by cold compress.

Referring to Figs. 2 to 7, the cold compress circulation device 10 includes a casing 100, a water reservoir 110, a cooler 120, an outlet pipe 130, a return pipe 140 and an ice box 150. Both the water reservoir 110 and the cooler 120 are disposed within the casing 100, and at least part of the ice box 150 is detachably disposed in the water reservoir 110. Both the outlet pipe 130 and the return pipe 140 are brought into fluidic communication with the water reservoir 110, and the cooler 120 is disposed on the outlet pipe 130 and/or the return pipe 140. The pack 20 is connected to the outlet pipe 130 and the return pipe 140 of the cold compress circulation device 10 by the connection pipe 21. It can be understood that the connection pipe 21 includes an outlet connection pipe and a return connection pipe. In practical use, water is circulated in a loop path leading from the water reservoir 110 sequentially through the outlet pipe 130, the outlet connection pipe in the connection pipes 21, the pack 20, the return connection pipe in the connection pipes 21 and the return pipe 140 back to the water reservoir 110. In some embodiments, the cooler 120 is arranged on the outlet pipe 130. Due to the presence of the ice box 150, water in the water reservoir 110 can be rapidly pre-cooled, and the pre-cooled water flows through, and is thus additionally cooled by, the cooler 120. The cooled water then enters pack 20 and experiences heat exchange there. In this way, effectively improved cooling efficiency can be achieved. In alternatively embodiments, the cooler 120 may be disposed on the return pipe 140, or on both the outlet pipe 130 and the return pipe 140. Depending on various pre-cooling requirements of practical applications, for example, on cooling rate or the like, the ice box 150 may be repeatedly taken out from the water reservoir 110 and frozen, for example, in a refrigerator, freezer or similar equipment, and the frozen ice box may be again disposed in the water reservoir. Of course, a user may replace the ice box 150 with a spare one that has been frozen in advance. Optionally, the ice box 150 may be designed as a disposable product in order to ensure hygiene and safety.

Optionally, the water reservoir 110 may include a water reservoir body 112 and an accommodating cavity 113 arranged at the bottom of the water reservoir body 112. At least part of the ice box 150 is detachably inserted in the accommodating cavity 113. Further, the accommodating cavity 113 includes a stopper 114, which is, for example, a recessed portion. An inner radial dimension of the stopper 114 is smaller than an outer radial dimension of the ice box 150. In this way, movement of the ice box 150 can be stopped. For example, during downward insertion of the ice box 150, the ice box 150 can be stopped from further downward movement which may lead to blockage of a passage between the return pipe 140 and the water reservoir 110. Optionally, the water reservoir 110 may be made of a polymer material. The stopper 114 may be integrally formed during blow molding. Alternatively, it is also possible to attach, after the accommodating cavity 113 has been formed, the stopper 114 to the accommodating cavity 113 by hot melting, gluing or the like. The stopper 114 may include, for example, several protrusions or ribs which are arranged oppositely, circumferentially or irregularly. Distances between these protrusions are smaller than the outer radial dimension of the ice box 150 so that, when the ice box 150 is disposed in the accommodating cavity 113, it is limited by the stopper 114 and will not further move. Fig. 8 shows an example of the stopper 114 with two protrusions. It should be understood that the stopper is not limited to being with two protrusions because it may have another number of protrusions and the protrusions may be otherwise shaped. The protrusions may be annularly arranged across the entire circumference of the ice box 150.

Preferably, the return pipe 140 is connected to the accommodating cavity 113, and there are gaps between the accommodating cavity 113 and the ice box 150, which bring the water reservoir body 112 into communication with the return pipe 140. Taking advantage of a temperature-specific gravity relationship of the liquid, arranging the accommodating cavity 113 at the bottom of the water reservoir body 112 (a lower portion in the direction of gravity) and inserting at least part of the ice box 150 in the accommodating cavity 113 result in a lower water temperature in the accommodating cavity 113 than in the water reservoir body 112. Moreover, as the return pipe 140 is first passed through the accommodating cavity 113, the return water can be pre-cooled as soon as possible. In practice, the return water circulated from the return pipe 140 flows back to the water reservoir body 112 through the gaps between the accommodating cavity 113 and the ice box 150. This allows an expanded heat exchange area between the return water and the ice box 150, resulting in additionally increased cooling efficiency. In one example, the accommodating cavity 113 may have an elliptic (as shown in Fig. 9), polygonal (as shown in Fig. 10) or otherwise shaped cross-sectional shape, while the ice box 150 has a circular cross-sectional shape. With this arrangement, the gaps will be created naturally as a result of the ice box 150 being inserted into the accommodating cavity 113. It should be understood that, here, by "gaps", it is not intended to limit the ice box 150 to being spaced from the accommodating cavity 113 over the entire circumference; rather, it should be appreciated that they only provide a communication access for the return water between the ice box 150 and the accommodating cavity 113. In some other embodiments, it is also possible, for example, that the ice box 150 is polygonal or assumes the shape of part of a circle while the accommodating cavity 113 is circular. A skilled person in the art can configure them appropriately.

Further, in some embodiments, the ice box 150 is filled with a cooling gel with a specific heat capacity greater than water. The greater specific heat capacity of the cooling gel than water means that it can store more cooling energy compared to the same volume of ice and can provide a better cooling effect. In order to achieve this purpose, a skilled person in the art can select a suitable material for the cooling gel, or choose another liquid with suitable heat exchange capabilities. In the latter case, the liquid may be sealed in the ice box in advance, and during use, the cold/hot compress circulation device as taught herein may be equipped with multiple such ice boxes. The user can use these ice boxes alternately over different periods of time, and each used ice box may be placed, for example, in a refrigerator to be prepared for subsequent use.

In one example, the cooler 120 includes a semiconductor cooling device 121 and a cooling fan 122. The semiconductor cooling device 121 includes a cooling surface and a heat dissipation surface. The cooling surface is thermally conductively connected to the outlet pipe 130 or the return pipe 140, and the cooling fan 122 is adapted to dissipate heat from the heat dissipation surface of the semiconductor cooling device 121. The semiconductor cooling device 121 has a simple, compact, inexpensive, reliable cooling structure, and using the semiconductor cooling device 121 in the cold/hot compress device to additionally cool the water that has been pre-cooled by the ice box 150 can result in an even better cooling effect. The cooling fan 122 may be fixed within the casing 100, and multiple heat dissipation holes (as shown in Fig. 1, not labeled) may be provided in a portion of the casing 100 corresponding to the cooling fan 122, in order to enable fast dissipation of heat from the heat dissipation surface of the semiconductor cooling device 121 to the outside of the casing 100. In some other embodiments, the cooler 120 may alternatively include a micro-compressor. In general terms, the micro-compressor is superior over the semiconductor cooling device in terms of both cooling capacity and efficiency. However, it would be bulky and structurally complex. A skilled person in the art can select an appropriate cooler and properly configure it according to the actual cooling capacity requirements of the pack. Other supporting components of the micro-compressor, such as cooling pipelines, heat exchange systems, etc., may be configured by a skilled person in the art in a conventional manner, and a further description thereof is omitted herein.

Referring to Fig. 4, optionally, the cold compress circulation device 10 may further include an air pump 180 and a water pump 160 both disposed within the casing 100. The air pump 180 and the water pump 160 are fluidically independent of each other. Preferably, the water pump 160 is disposed between the cooler 120 and the water reservoir 110. For ease of use, the cold compress circulation device 10 may use the water pump 160 to further drive the circulation of the cooling water and use the air pump 180 to provide pressurized air to the pack. Optionally, an inlet port of the water pump 160 may be arranged at the bottom of the water reservoir body 112 in order to enable the cooler water in the water reservoir body 112 to be pumped out. This can result in an increase in cooling efficiency of the overall cold compress circulation system. The water pump 160 may be implemented as, for example, a centrifugal pump disposed under the water reservoir body 112 in the vertical direction. Additionally, an inlet port of the water pump 160 may be oriented vertically and directly open at the bottom of the water reservoir body 112, and an outlet port of the water pump 160 may be oriented horizontally and connected by a pipe to the cooler 120 and hence to the outlet pipe 130, or connected by a pipe directly to the outlet pipe 130.

With the above arrangement, for example, of the water reservoir 110, the ice box 150, the cooler 120, the water pump 160 and the air pump 180, the cold compress circulation device provides high cooling efficiency, is compact and portable and can be made at lower cost. Therefore, it is suitable for use both in healthcare institutions and in household applications and will be thus more commercially successful than the traditional ones that are limited to use for healthcare. In order to achieve higher cooling efficiency, existing cold compress circulation devices are usually equipped with a large water reservoir that can store more water. This makes the conventional devices bulky, heavy, expensive and thus unsuitable for use in household applications.

Optionally, the cold compress circulation device 10 may further include pipe interfaces 170. In particular, the pipe interfaces 170 may include an outlet interface and a return interface, both disposed on the casing 100. The outlet interface is connected to the outlet pipe 130, and the return interface is connected to the return pipe 140. The pipe interfaces 170 can facilitate connection of the connection pipe 21 for the pack 20. The pipe interfaces 170 may be implemented as, for example, quick connect/disconnect interfaces which allow easy mating and unmating by the user. In some embodiments, the pack 20 is detachably connected to the connection pipe 21. In these embodiments, the pack 20, the connection pipe 21 and the cold compress circulation device 10 can be separated from one another and easily assembled together, resulting in enhanced portability. In some other embodiments, the pack 20 is fixed to the connection pipes 21, and they are handled together as a single part. In such embodiments, the device can be made ready for use simply by connecting the connection pipe 21 to the pipe interfaces 170, and interfaces on the side of the pack 20 are dispensed with, resulting in increased reliability. Of course, in still some other embodiments, it also possible that the pack 20, the connection pipe 21 and the cold compress circulation device 10 cannot be separated apart once they are assembled together. This can dispense with the use of the interfaces, resulting in even improved reliability of the pipe.

In some embodiments, the pipe interfaces 170 may include an outlet interface, a return interface and a pressurization interface, which are all disposed on the casing 100, as shown in Fig. 6. In such embodiments, the cold compress circulation device may supply a gas to the pack 20 through the air pump 180 and the pressurization interface, which pressurizes the pack 20 and enables it to better fit the diseased part, resulting in an even better therapeutic effect. Preferably, the pressurization interface is disposed between the outlet and return interfaces. This is favorable to independent circulation and control of liquid and gas fluids.

Optionally, as shown in Fig. 7, the water reservoir 110 defines an ice box receptacle 111 that can be opened and closed. At least part of the ice box 150 can be inserted into the water reservoir 110 through the ice box receptacle 111. Preferably, at the top of the casing 100 of the cold compress circulation device, a fluid injection port is provided, which provides an access to the water reservoir 110 and is in alignment or coincidence with the ice box receptacle 111. The ice box 150 is detachably arranged in the water reservoir through the fluid injection port on the casing 100 or the ice box receptacle 111 in the water reservoir 110. In some other embodiments, the casing 100 and/or water reservoir 110 may be provide with a lid that can be opened by removing it or pushing or pulling it aside, and the fluid injection port or the ice box receptacle 111 is not included. In these embodiments, the ice box 150 can be inserted into the water reservoir 110, after the lid is removed or pushed or pulled aside.

Furthermore, the casing 100 may further include a display screen 191, and a handle 192 on the top of the casing 100. The display screen 191 may be adapted, for example, to display information such as real-time temperatures or flow rates of the cooling water at the outlet and return interfaces. As another example, it may be adapted to display information such as an air pressure at the pressurization interface. The handle 192 can enhance portability of the cold compress circulation device.

In the foregoing embodiments, water from the water reservoir 110 flows through the water pump 160, the cooler 120 and the outlet pipe 130 into the pack. In some embodiments, in order to enable water from the water reservoir 110 to enter the pack within a shorter time, it flows into the pack through only the water pump 160 and the outlet pipe 130. Moreover, after exiting the pack, it flows through the return pipe 140 and cooler 120 back to the water reservoir 110.

In summary, the present invention provides a cold compress circulation device and a cold compress circulation system. The cold compress circulation device includes a casing, a water reservoir, a cooler, an outlet pipe, a return pipe and an ice box. Both the water reservoir and the cooler are disposed inside the casing, and at least part of the ice box is detachably disposed in the water reservoir. Both the outlet and return pipes are brought into fluidic communication with the water reservoir, and the cooler is disposed on the outlet pipe and/or the return pipe. In this arrangement, the detachable ice box serves as a means for accelerated cooling and can be conveniently replaced by a user. Moreover, the ice box does not lead to an increased amount of water to be circulated or release small ice piece when heated, which may lead to reduced cooling efficiency. Thus, both enhanced cooling efficiency and increased convenience of use are achieved for the user.

The description presented above is merely that of a few preferred embodiments of the present invention and is not intended to limit the scope thereof in any sense. Any and all changes and modifications made by those of ordinary skill in the art based on the above teachings fall within the scope as defined in the appended claims.

## Claims

1. A cold compress circulation device, comprising a casing, a water reservoir, a cooler, an outlet pipe, a return pipe and an ice box,
wherein both the water reservoir and the cooler are arranged in the casing, at least part of the ice box is detachably arranged in the water reservoir, both the outlet pipe and the return pipe are in fluidic communication with the water reservoir, and the cooler is arranged on the outlet pipe and/or the return pipe.

2. The cold compress circulation device according to claim 1, wherein the water reservoir comprises a water reservoir body and an accommodating cavity at the bottom of the water reservoir body, and wherein the at least part of the ice box is detachably inserted into the accommodating cavity.

3. The cold compress circulation device according to claim 2, wherein the accommodating cavity comprises a stopper having an inner radial dimension that is smaller than an outer radial dimension of the ice box in order to limit a movement of the ice box.

4. The cold compress circulation device according to claim 3, wherein the stopper comprises at least two protrusions arranged circumferentially, and wherein any two of the protrusions are separated apart by a distance that is smaller than the outer radial dimension of the ice box.

5. The cold compress circulation device according to claim 3, wherein the stopper comprises an annular protrusion extending along an entire circumference of the ice box, and wherein the protrusion has an inner radial dimension that is smaller than the outer radial dimension of the ice box.

6. The cold compress circulation device according to any one of claims 2 to 5, wherein the return pipe is connected to the accommodating cavity, a gap is formed between the accommodating cavity and the ice box, and the water reservoir body is brought into communication with the return pipe by the gap.

7. The cold compress circulation device according to claim 6, wherein the accommodating cavity has an elliptic or polygonal cross section, and the ice box has a circular cross section.

8. The cold compress circulation device according to any one of claims 1 to 7, wherein the ice box encloses therein a cooling gel or another liquid configured for heat exchange, and the cooling gel has a specific heat capacity greater than water.

9. The cold compress circulation device according to any one of claims 1 to 8, wherein the cooler comprises a semiconductor cooling device and a cooling fan, the semiconductor cooling device comprising a cooling surface and a heat dissipation surface, wherein the cooling surface is thermally conductively connected to the outlet pipe or the return pipe, and the cooling fan is configured to dissipate heat from the heat dissipation surface of the semiconductor cooling device.

10. The cold compress circulation device according to claim 9, wherein heat dissipation holes are provided in a portion of the casing corresponding to the cooling fan.

11. The cold compress circulation device according to any one of claims 1 to 10, wherein the cooler comprises a micro-compressor.

12. The cold compress circulation device according to any one of claims 1 to 10, wherein a fluid injection port is provided on top of the casing to provide an access to the water reservoir, and wherein the ice box is inserted into the water reservoir through the fluid injection port.

13. The cold compress circulation device according to any one of claims 1 to 10, wherein the casing comprises a lid which can be opened by removing the lid or pushing or pulling the lid aside, and wherein the ice box is inserted into the water reservoir when the lid is removed or pushed or pulled aside.

14. The cold compress circulation device according to any of claims 2 to 13, further comprising an air pump and a water pump both arranged in the casing, the air pump and the water pump fluidically independent of each other, the water pump comprising an inlet port arranged at the bottom of the water reservoir body, the inlet port of the water pump oriented vertically and directly connected to the bottom of the water reservoir body, the water pump having an outlet port oriented horizontally and connected to the cooler or the outlet pipe.

15. The cold compress circulation device according to claim 14, further comprising an outlet interface, a return interface and a pressurization interface, all arranged on the casing, the outlet interface connected to the outlet pipe, the return interface connected to the return pipe, the pressurization interface connected to the air pump.

16. The cold compress circulation device according to claim 15, wherein the pressurization interface is disposed between the outlet interface and the return interface.

17. The cold compress circulation device according to any of claims 1 to 16, wherein the ice box is disposable or reusable and encloses therein a liquid configured for heat exchange.

18. A cold compress circulation system, comprising the cold compress circulation device according to any one of claims 1 to 17 and a pack, the pack brought into fluidic communication with the cold compress circulation device by a connection pipe.

19. The cold compress circulation system according to claim 18, wherein the pack is detachable or fixedly connected to the connection pipe.

20. The cold compress circulation system according to claim 18, wherein at least two of the pack, the connection pipe and the cold compress circulation device are integral or pluggable with each other.
